# EUROPÄISCHE PATENTSCHRIFT

(11) **EP 3 395 723 B1**
(45) Veröffentlichungstag und Bekanntmachung des Hinweises auf die Patenterteilung: **06.10.2021**
(21) Anmeldenummer: 18161799.4
(22) Anmeldetag: 14.03.2018
(51) Int. Cl.: B65D 90/48, G01B 5/06, G01B 5/00

(54) **FAHRRADTRANSPORTBEHÄLTER SOWIE SCHRITTHÖHEN-MESSSYSTEM**
BICYCLE TRANSPORT CONTAINER AND STEP HEIGHT MEASUREMENT SYSTEM
RÉCIPIENT DE TRANSPORT DE BICYCLETTE AINSI QUE SYSTÈME DE MESURE DE HAUTEUR DE MARCHE

(30) Priorität: 05.04.2017 DE 202017001816 U
(43) Veröffentlichungstag der Anmeldung: 31.10.2018
(73) Patentinhaber: Canyon Bicycles GmbH, 56073 Koblenz (DE)
(72) Erfinder: KEILLER, John, Manchester M1 3NJ (GB)
(74) Vertreter: dompatent von Kreisler Selting Werner - Partnerschaft von Patent- und Rechtsanwälten mbB

(56) Entgegenhaltungen:
- DE-U1- 29 516 898
- FR-A1- 2 729 123
- JP-A- H07 215 389
- JP-A- 2013 212 875
- JP-U- S58 136 104
- TW-U- M 400 997
- US-A- 3 929 225
- US-A- 5 491 907
- US-A1- 2017 066 588
- US-B1- 6 226 881

## Beschreibung

Die Erfindung betrifft einen Fahrradtransportbehälter, insbesondere einen Fahrradkarton mit einem Schritthöhen-Messsystem, das in den Fahrradtransportbehälter integriert ist.

Zum Transport, insbesondere zum Versand von Fahrrädern sind Fahrradtransportbehälter, insbesondere Fahrradtransportkartons bekannt. Um einen Transport mit Transportunternehmen wie DHL oder UPS zu ermöglichen, müssen derartige Transportbehälter eine definierte hohe Stabilität aufweisen und dürfen eine Maximalgröße nicht überschreiten. Ein hierfür geeigneter Fahrradtransportbehälter ist beispielsweise in EP 2 239 210 beschreiben. Mit diesem Transportbehälter können Fahrradrahmen in teilweise vormontierten Zustand zusammen mit den nicht montierten Rädern zuverlässig versandt werden. Es treten hierbei bei herkömmlicher Handhabung des Fahrradtransportbehälters beim Transport keine Beschädigungen an dem Fahrrad auf.

Der in EP 2 239 210 beschriebene Fahrradtransportbehälter weist einen Außenbehälter bzw. Außenkarton auf. In diesem sind speziell ausgebildete Aufnahmeelemente, die vorzugsweise ebenfalls aus Karton hergestellt sind, für den Hinterbau bzw. die Fahrradgabel angeordnet. Die beiden Aufnahmeelemente sind auf einem Bodenelement des Außenbehälters angeordnet. Zur Entnahme des vormontierten Fahrradrahmens sowie der beiden Räder muss der Außenbehälter an seiner Oberseite geöffnet werden, so dass ein Entnehmen des vormontierten Fahrradrahmens sowie der Räder nach oben erfolgen kann. Dies stellt bei Sporträdern mit entsprechend geringem Gewicht keinerlei Problem dar. Bei einer Verwendung eines entsprechenden Fahrradtransportbehälters für schwerere Fahrräder, wie beispielsweise E-Bikes, ist eine Entnahme nach oben jedoch nachteilig.

Eine weitere grundsätzlich von der Art des Fahrradtransportbehälters und generell von den beim Transport auftretenden Problemen unabhängiges Problem ist das Einstellen der Sattelhöhe. Die Sattelhöhe soll insbesondere in Abhängigkeit der Schritthöhe eingestellt werden, so dass beim Treten einerseits ein annähernd vollständiges Durchstrecken des Beins gewährleistet ist, andererseits ein seitliches Hin- und Herbewegen bzw. Hin- und Herrutschen des Gesäßes auf dem Sattel vermieden ist. Dokument US 3 929 225 A offenbart den Oberbegriff des Anspruchs 1.

Aufgabe der Erfindung ist es, einen Fahrradtransportbehälter, insbesondere einen Fahrradtransportkarton mit einem einfach handhabbaren Schritthöhen-Messsystem bereitzustellen, dessen Handhabung verbessert ist.

Die Lösung der Aufgabe erfolgt erfindungsgemäß durch die Merkmale des Anspruchs 1.

Das erfindungsgemäße Fahrradtransportbehälter ist mit einem Schritthöhen-Messsystem, integriert und weist ein Basiselement auf. In dem Basiselement ist ein vertikal verlaufender Schlitz vorgesehen. Zumindest auf einer Seite, das heißt links oder rechts neben dem Schlitz ist auf einer Oberfläche des Basiselements eine Sitzhöhenskala angeordnet. Eine korrespondierende Skala kann insbesondere auf der den Sattel tragenden Sattelstütze vorgesehen sein. Diese Skala steht in der Relation zu der Skala auf dem Basiselement. Ferner weist das erfindungsgemäße Schritthöhen-Messsystem ein Messelement auf. Das Messelement weist einen Führungs-Ansatz auf, der in den Schlitz einsteckbar ist, so dass das Messelement vertikal in dem Basiselement verschoben werden kann. Ferner weist das Messelement einen mit dem Führungselement verbundenen Messansatz zum Messen der Schritthöhe auf. Zum Messen der Schritthöhe eines Benutzers wird der Führungsansatz in den Schlitz des Basiselements eingeführt, so dass der Messansatz des Messelements zwischen den Beinen des Benutzers angeordnet ist. Zum Messen der Schritthöhe wird der Messansatz sodann zwischen den Beinen des Benutzers so weit nach oben wie möglich geführt. Mit dieser Stellung kann sodann mit Hilfe der Sitzhöhenskala eine Sitzhöhe bestimmt werden. Hierbei kann die Sitzhöhenskala eine Zahl oder Bezeichnung sein, die mit der entsprechenden Zahl oder Bezeichnung auf der Sattelstütze korrespondiert bzw. in Relation steht.

In bevorzugter Weiterbildung sind auf beiden Seiten des Schlitzes unterschiedliche Skalen angeordnet. Diese sind von den unterschiedlichen verwendeten Reifengrößen abhängig.

Bei einer besonders bevorzugten Weiterbildung des Sitzhöhen-Messsystems ist das Basiselement durch Knicken und/oder Zusammenfügen eines ebenen Zuschnittselements hergestellt. Bei einem ebenen Zuschnittelement handelt es sich insbesondere um einen Zuschnittkarton.

Bevorzugt ist es ferner, dass das Basiselement ein Bodenelement und ein insbesondere zweiteilig ausgebildetes vorzugsweise mit dem Bodenelement verbundenes Stirnseitenelement aufweist. Hierbei ist es insbesondere bei einer zweiteiligen Ausgestaltung des Stirnseitenelements bevorzugt, dass der Schlitz zwischen den beiden Teilen des Stirnseitenelements angeordnet bzw. durch diese ausgebildet ist. Insbesondere ist es möglich durch ein entsprechendes Umknicken der beiden Teile des Stirnseitenelements nach innen eine nach innen weisende Führungsfläche in dem Schlitz zu erzeugen, die breiter als die Dicke des Materials des Stirnseitenelements selbst ist.

Des Weiteren ist es bevorzugt, dass das Basiselement ein, insbesondere zwei Seitenelemente aufweist. Diese sind vorzugsweise mit dem Bodenelement und/oder dem mindestens einen Stirnseitenelement verbunden und besonders bevorzugt einstückig ausgebildet. Insbesondere ist je Seite ein Seitenelement vorgesehen, das jeweils mit dem Bodenelement und einem der beiden Stirnseitenelemente verbunden ist.

Bei einer besonders bevorzugten Ausführungsform des Schritthöhen-Messsystems ist das Messelement aus dem Basiselement heraustrennbar. Insbesondere wenn das Basiselement aus Karton besteht, ist es durch Vorsehen einer entsprechen Perforation auf einfache Weise möglich, ein heraustrennbares Messelement vorzugsehen.

In bevorzugter Weiterbildung weist das Messelement zusätzlich ein Anschlagelement auf. Dieses ist ebenfalls in bevorzugter Ausführungsform aus dem Basiselement heraustrennbar. Des Weiteren ist es bevorzugt, dass das Anschlagelement durch Zusammenfügen, insbesondere Zusammenstecken mit dem Führungsansatz und/oder dem Messansatz mit diesem verbindbar ist. Insbesondere entsteht hierdurch im Querschnitt ein Kreuz. Das Anschlagelement ist hierbei derart angeordnet, dass auf der einen Seite des Anschlagelements der Führungsansatz und auf der anderen Seite der Messansatz vorsteht. Das Einführen des Führungsansatzes in den Schlitz kann hierbei nur derart weit erfolgen, bis das Anschlagelement an einer Außenseite des Basiselements, insbesondere an der Außenseite des mindestens einen Stirnseitenelements anliegt. Des Weiteren ist es bevorzugt, dass das Anschlagelement flächig an der entsprechenden Außenseite anliegt, so dass hierdurch gewährleistet ist, dass eine Oberkante des Messansatzes horizontal verläuft. Hierdurch ist die Messgenauigkeit verbessert. Des Weiteren kann eine Oberseite des Anschlagelements derart angeordnet und ausgebildet sein, dass es hierdurch möglich ist auf einfache Weise die Messscala abzulesen.

Der Führungsansatz und der Messansatz sind vorzugsweise einstückig ausgebildet.

Der erfindungsgemäße Fahrradtransportbehälter, der insbesondere als Fahrradtransportkarton hergestellt ist, ist vorzugsweise wie nachstehend beschrieben ausgebildet. Er weist einen Außenbehälter auf, bei dem es sich vorzugsweise um einen aus Karton hergestellten Außenkarton handelt. Innerhalb des Außenbehälters ist das Basiselement angeordnet. Das Basiselement dient zum Halten eines Fahrradrahmens. Insbesondere bevorzugt ist, dass der Fahrradrahmen derart teilweise vormontiert ist, dass das Hinterrad bereits montiert ist. Vorzugsweise weist das Basiselement ein Bodenelement auf. Das Bodenelement liegt auf einem Bodenteil des Außenbehälters auf. Bevorzugt ist es hierbei, dass das Basiselement ein durchgehendes Bodenelement aufweist, so dass eine insbesondere flächige Aufnahme des Bodenelements an dem Bodenteil des Außenbehälters, das heißt auf der Innenseite des Bodenteils des Außenbehälters vorgesehen ist. Insbesondere sind die Außenabmessungen des Bodenteils hierbei derart gewählt, dass sie zumindest teilweise der inneren Breite sowie der inneren Länge des Außenbehälters entsprechen. Hierdurch ist ein Verrutschen des Bodenelements in dem Außenbehälter während des Transports vermieden. Bevorzugt ist hierbei insbesondere aus Stabilitätsgründen, dass das entsprechende Bodenteil des Außenbehälters eine rechteckigen Querschnitt aufweist, so dass das Bodenelement vorzugsweise über die gesamte Länge und vorzugsweise auch über die gesamte Breite an der Innenseite des Außenbehälters anliegt.

Des Weiteren ist der insbesondere quaderförmig ausgebildete Außenbehälter derart ausgebildet, dass er ein öffenbares Seitenteil aufweist. Vorzugsweise handelt es sich hierbei bei einem quaderförmigen Außenbehälter um das schmalere Seitenteil. Das schmalere Seitenteil kann beispielsweise entsprechend einer Tür geöffnet werden. Das Seitenteil, bei dem es sich insbesondere um das Seitenteil der Stirnseite handelt, ist hierbei entlang einer vertikalen Längskante mit einem der beiden größeren in Längsrichtung des Kartons verlaufenden Seitenteile verbunden. Hierbei handelt es sich insbesondere um eine Knickkante des Kartons. Auf der gegenüberliegenden Seite ist durch eine entsprechende Lasche, ein Klebeband oder dergleichen ein Fixieren des öffenbaren Seitenteils während des Transports möglich. Durch Öffnen des Seitenteils ist es sodann möglich, das Basiselement zusammen mit dem von diesem gehaltenen bzw. getragenen Fahrrad seitlich aus dem Außenbehälter herauszuziehen. Das Basiselement entspricht sodann einer Art Plattform, auf der das insbesondere vormontierte Fahrrad angeordnet ist. Ein Herausnehmen des Fahrrads nach oben aus dem Außenbehälter ist nicht mehr erforderlich.

Insbesondere ist das vormontierte Fahrrad auf dem Basiselement derart angeordnet, dass das Fahrrad zwar einerseits von dem Basiselement gehalten ist, andererseits zumindest überwiegend von dem Basiselement nicht umgeben ist. Sobald das Basiselement zusammen mit dem Fahrrad aus dem Außenbehälter herausgezogen ist, ist es somit auf einfache Weise möglich, das Fahrrad seitlich vom Basiselement herunter zu nehmen. Hierdurch ist ein einfaches Entnehmen auch schwererer Fahrräder aus dem Außenbehälter möglich. Selbstverständlich ist dies auch bei leichten Fahrrädern vorteilhaft.

In bevorzugter Weiterbildung der Erfindung weist das Basiselement eine Ausnehmung zu Aufnahme des insbesondere vormontierten Hinterrades auf. Die Ausnehmung ist hierbei insbesondere schlitzförmig und verläuft im Wesentlichen in Längsrichtung des Basiselements. Das Hinterrad kann somit zum Transport von oben in die Ausnehmung gestellt werden. Insbesondere weist die Ausnehmung hierbei eine Höhe von 4 cm bis 8 cm auf, so dass das Hinterrad sicher in der Ausnehmung gehalten ist.

Des Weiteren ist es bevorzugt, dass das Element eine Ausnehmung zur Aufnahme der Gabelrohre aufweist. Hierbei kann es sich um zwei gesonderte Ausnehmungen oder eine gemeinsame Ausnehmung für beide Gabelrohrenden handeln. Die Gabelrohrenden werden hierbei zum Transport in die vorzugsweise zwei Ausnehmungen von oben eingesteckt und sind hierdurch in ihrer Lage fixiert. Die Gabelrohrenden werden hierbei vorzugsweise wiederum 4 cm bis 8 cm in die Ausnehmung eingesteckt, so dass ein sicheres Halten der Gabelrohrenden in dem Basiselement gewährleistet ist. Vorzugsweise sind die beiden Ausnehmungen für die Gabelrohrenden in Längsrichtung des Basiselements im Wesentlichen hintereinander angeordnet. Zum Transport erfolgt ein Verdrehen der Vorderradgabel zur herkömmlichen Ausrichtung um 90°, so dass der ebenfalls vorzugsweise vormontierte Fahrradlenker im Wesentlichen parallel zur Rahmenlängsrichtung verläuft.

Des Weiteren ist es bevorzugt, eine Ausnehmung zur Aufnahme eines nicht montierten Vorderrades im Basiselement vorzusehen. Hierbei handelt es sich wiederum vorzugsweise um eine schlitzförmige Ausnehmung, die insbesondere in Längsrichtung des Basiselements verläuft. Die Höhe der Ausnehmung beträgt wiederum 4 cm bis 8 cm, so dass auch das Vorderrad sicher in der Ausnehmung angeordnet werden kann. Insbesondere ist die Ausnehmung für das Vorderrad neben den Ausnehmungen für die Gabelrohrenden angeordnet, so dass zum Transport das Vorderrad seitlich neben einem Oberrohr sowie einem Unterrohr des Fahrradrahmens angeordnet ist. Zur Stabilisierung kann das Vorderrad ferner mit dem Fahrradrahmen über beispielsweis als Band ausgebildete Halteelemente verbunden sein.

Bei einer weiteren bevorzugten Ausführungsform weist das Basiselement insbesondere zwei zumindest teilweise seitlich neben dem vormontierten Hinterrad oder neben dem Hinterbau des Fahrradrahmens angeordnete Seitenelemente auf. Die Seitenelemente sind hierbei vorzugsweise einstückiger Bestandteil des Basiselements, wobei das Basiselement vorzugsweise aus einem ebenen Zuschnittelement durch Knicken und Zusammenfügen hergestellt ist. Insbesondere handelt es sich um einen Zuschnittkarton, aus dem das Basiselement hergestellt ist. Die Seitenelemente sind hierbei vorzugsweise derart angeordnet, dass sie einen Abstand zueinander aufweisen, welcher der inneren Breite des Außenbehälters entspricht. Die Seitenelemente liegen somit zum Transport an der Innenseite der Seitenelemente des Außenbehälters an. Beim Herausziehen des Basiselements aus dem Ausbehälter gleiten die Seitenelemente somit an der Innenseite der Seitenelemente des Außenbehälters entlang. Durch derartig angeordnete Seitenelemente ist eine weitere Versteifung des Transportbehälters realisiert.

Es ist daher des Weiteren bevorzugt, dass zwei einander gegenüberliegende Seitenelemente vorgesehen sind, die zusätzlich zur Versteifung mit zumindest einem Stirnseitenelement verbunden sind. Das Stirnseitenelement weist wiederum eine Breite auf, die der inneren Breite des Außenbehälters entspricht und liegt beim Transport an dem Stirnseitenelement des Außenbehälters an dessen Innenseite an. Besonders bevorzugt ist es, dass ein Herausziehen des Basiselements aus dem Außenbehälter durch Greifen des Stirnseitenelements erfolgt. Hierzu kann das Stirnelement Greifausnehmungen aufweisen.

Bei einer weiteren bevorzugten Ausführungsform ist zur weiteren Versteifung ein Verbindungselement vorgesehen. Das Verbindungselement ist vorzugsweise ebenfalls aus Karton hergestellt. Insbesondere handelt es sich um einen im Wesentlichen als quaderförmiges Aufnahmeelement ausgebildetes Verbindungselement. In diesem können Kleinteile wie Fahrradpedale, Werkzeuge etc. zum Transport angeordnet sein. Das Verbindungselement ist vorzugsweise derart angeordnet, dass es mit den beiden Seitenelementen verbunden ist. Insbesondere weist das Verbindungselement wiederum eine Breite auf, die der inneren Breite des Außenbehälters entspricht. Des Weiteren ist es bevorzugt, dass das Verbindungselement zusätzlich mit dem zumindest einen Stirnseitenelement verbunden ist. Hierdurch ist insbesondere zum Herausziehen des Basiselements aus dem Außenbehälter ein in sich stabiles Basiselement realisiert.

Bei einer weiteren bevorzugten Ausführungsform des Fahrradtransportbehälters ist zusätzlich ein Haltelement vorgesehen. Das Halteelement weist wiederum in bevorzugter Ausführungsform eine Breite auf, die der inneren Breite des Außenbehälters entspricht. Das Haltelement ist vorzugsweise im Bereich der Fahrradgabel beziehungsweise im Bereich eines mit der Fahrradgabe verbundenen Fahrradlenkers angeordnet. Durch das Halteelement erfolgt somit ein Fixieren des insbesondere vormontierten Fahrradrahmens in dessen vorderem Bereich innerhalb des Außenbehälters. Hierzu weist in bevorzugter Weiterbildung das Halteelement eine Ausnehmung zur Aufnahme des Lenkers auf. Des Weiteren ist es möglich, dass das Halteelement eine insbesondere schlitzförmige Ausnehmung zur Aufnahme des Vorderrades aufweist. Diese schlitzförmige Ausnehmung ist sodann zum Transport des Fahrrads vorzugsweise gegenüber der schlitzförmigen Ausnehmung für das Vorderrad im Basiselement angeordnet. Insbesondere betrifft die Erfindung einen Fahrradtransportbehälter zusammen mit in dem Außenbehälter angeordneten vom Basiselement gehaltenen Fahrradrahmen. Insbesondere handelt es sich nicht nur um einen Fahrradrahmen, sondern um ein zumindest teilweise vormontiertes Fahrrad.

Das erfindungsgemäße Schritthöhen-Messsystem mit einem Messelement, das in einen Schlitz eingesteckt werden kann, kann alternativ auch bei Fahrradtransportbehältern vorgesehen sein, die nicht wie vorstehend beschrieben ausgebildet sind. Ferner kann es sich auch um einen herkömmlichen quaderförmigen Karton als Fahrradtransportbehälter handeln, der in einer Außenseite eines Stirnelements einen Schlitz aufweist oder in den in einer Außenseite insbesondere eines Stirnelements ein Schlitz auf einfache Weise eingebracht werden kann. Auch die insbesondere aus einem Teil des Fahrradtransportbehälters, insbesondere des Fahrradtransportkartons heraustrennbaren Teile des Messelements können auch aus einer Seitenwand eines herkömmlichen Fahrradtransportbehälters herausgetrennt werden.

Nachfolgend werden die Erfindungen anhand einer bevorzugten Ausführungsform unter Bezugnahme auf die anliegenden Zeichnungen näher erläutert.

Es zeigen:
- Fig. 1: eine schematische Seitenansicht eines Fahrradtransportbehälters mit teilweise herausgezogenem Basiselement,
- Fig. 2: eine schematische Seitenansicht des vollständig aus dem Außenbehälter herausgezogenen Basiselements,
- Fig. 3: eine schematische Ansicht des Stirnseitenelements in Richtung des Pfeils III in Fig. 2,
- Fig. 4: eine schematische Schnittansicht entlang der Linie IV-IV in Fig. 3.
- Fig. 5: eine schematische Draufsicht des Zuschnittkartons des Basiselements,
- Fig. 6: eine schematische Draufsicht eines Messelements,
- Fig. 7: eine schematische Unteransicht eines Halteelements und
- Fig. 8: eine schematische Draufsicht eines das Hinterrad aufnehmende Innenteils.

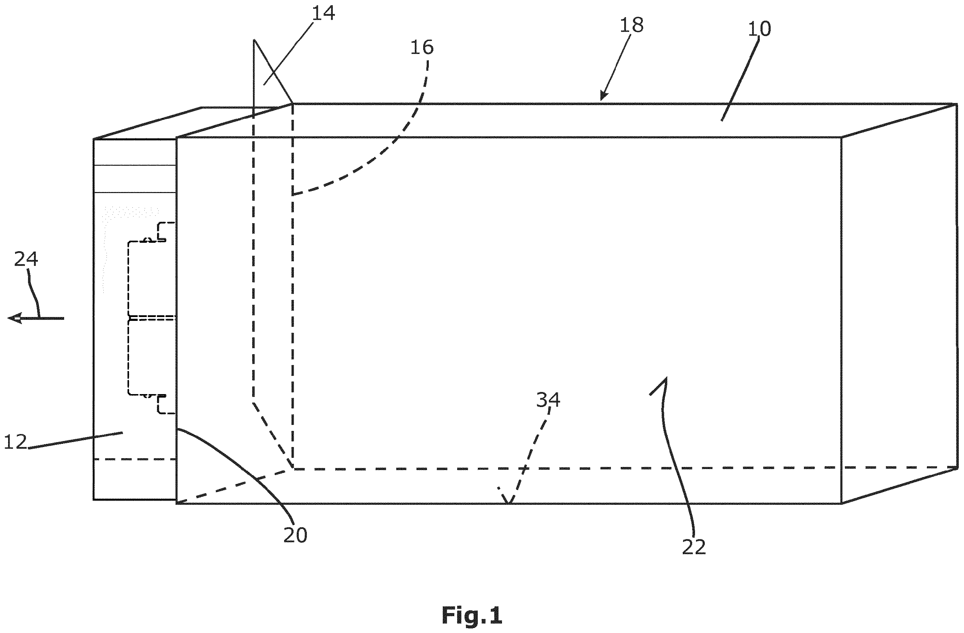

In einem Außenbehälter, bei dem es sich insbesondere um einen Außenkarton 10 handelt, ist ein Basiselement 12 angeordnet, das im dargestellten Ausführungsbeispiel einen vormontierten Fahrradrahmen sowie ein Vorderrad aufnimmt. Bei dem Außenkarton handelt es sich um einen quaderförmigen Karton mit einem Seitenteil 14, das entsprechend einer Tür öffenbar ist. Das Seitenteil 14 ist an einer Knickkante 16 mit einem sich in Längsrichtung erstreckenden Seitenteil 18 verbunden. Bei geschlossenem Karton ist das Seitenteil 14 mit einer Kante 20 des in Fig. 1 vorderen Seitenteils 22 zum Schließen des Kartons verbunden. Das Schließen kann hierbei über entsprechend übliche Laschen und dergleichen erfolgen. Ebenso kann auch an der Kante 20 ein weiteres Seitenteil vorgesehen sein, so dass anschließend zwei sich überdeckende Seitenteile 14 angeordnet sind.

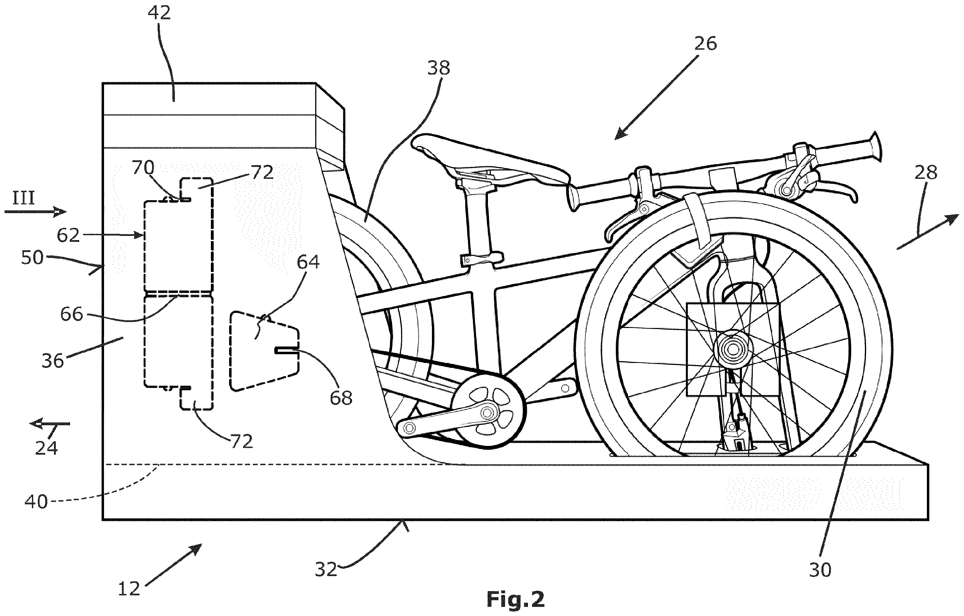

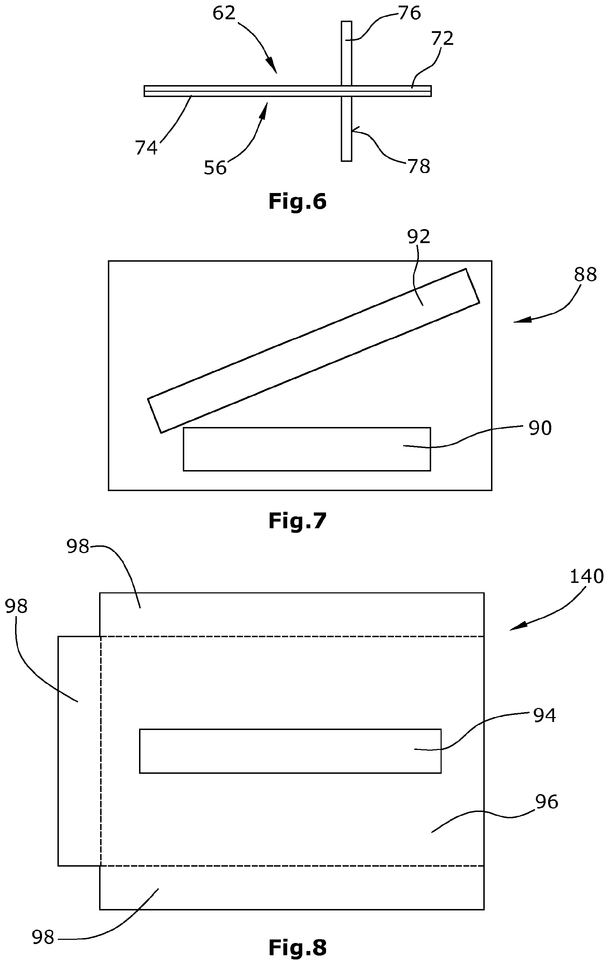

Das das vormontierte Fahrrad tragende Basiselement 12 kann in Längsrichtung 24 aus dem Außenkarton 10 herausgezogen werden. Nach dem Herausziehen ist das Basiselement wie in Fig. 2 dargestellt angeordnet, so dass ein Fahrradrahmen 26 mit vormontiertem Hinterrad, vormontierter Gabel und vormontiertem Lenker auf einfache Weise aus dem Basiselement in Richtung eines Pfeils 28 entnommen werden kann. Zuvor wird gegebenenfalls ein über einen Klettverschluss oder ein anderes Element mit dem Rahmen verbundenes Vorderrad 30 aus dem Basiselement 12 entnommen. Ferner muss zuvor von dem Lenker ein aus Übersichtlichkeitsgründen nicht dargestellter als Halteelement dienender Karton (Fig. 7) abgenommen werden.

Im dargestellten Ausführungsbeispiel weist das Basiselement 12 ein Bodenelement 32 auf. Das Bodenelement 32 erstreckt sich über die gesamte Länge des Außenkarton 10 und gleitet beim Herausziehen in Längsrichtung 24 auf einem Bodenteil 34 des Außenkartons 10. Die Außenabmessungen des Bodenelements 32 entsprechen hierbei den Innenabmessungen des Außenkarton 10, so dass ein Verrutschen des Basiselements im Außenkarton 10 während des Transports vermieden ist.

Des Weiteren weist das Bodenelement im dargestellten Ausführungsbeispiel zwei Seitenelemente 36 auf. Die beiden Seitenelemente 36 sind, wenn das Basiselement wie in Fig. 2 dargestellt entsprechend zusammengefaltet ist, einander gegenüberliegend angeordnet, wobei ein Hinterrad 38 zumindest teilweise zwischen den beiden Seitenelementen 36 angeordnet ist. Zwischen dem Hinterrad 36 bzw. einem Hinterbau des Fahrradrahmens und den Innenseiten der Seitenelemente 36 können Abstandshalter, Versteifungselemente, Pufferelemente oder dergleichen angeordnet werden, um ein sicheres Halten des Hinterrads in diesem Bereich zu gewährleisten. Ebenso kann in einem unteren Bereich zwischen den beiden Seitenelementen 36 ein zusätzliches Kartonelement wie durch die gestrichelte Linie 40 dargestellt, vorgesehen sein. Dieses Kartonelement kann eine sich in Längsrichtung 24 erstreckende Ausnehmung aufweisen, in der das Hinterrad 38 angeordnet und somit ebenfalls seitlich fixiert ist.

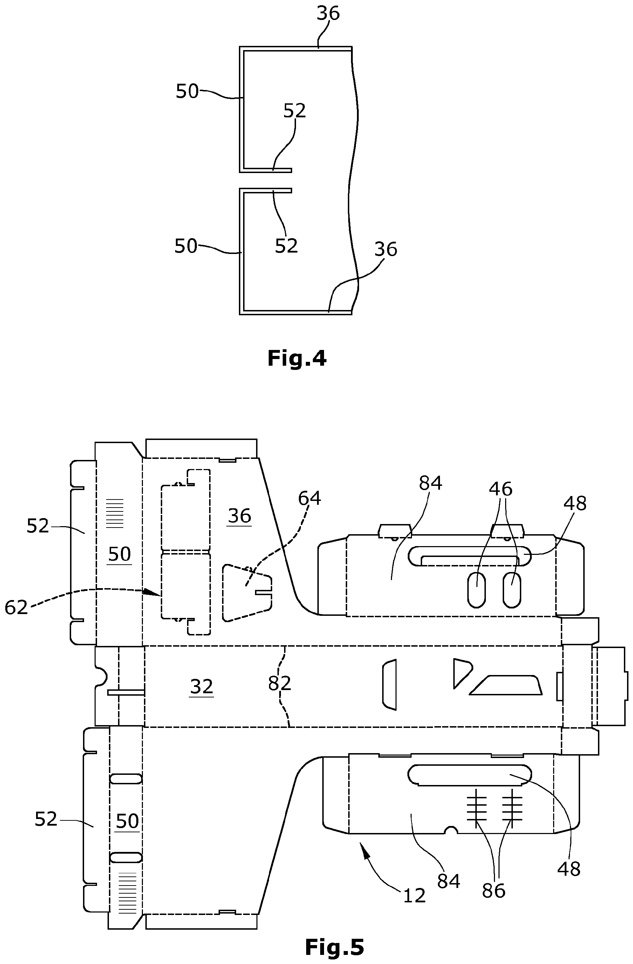

Die beiden Seitenelemente 36 sind über ein Verbindungselement 42 miteinander verbunden. Im dargestellten Ausführungsbeispiel handelt es sich bei dem Verbindungselement 42 um einen quaderförmigen Karton, in dem beispielsweise die Pedale, Werkzeug zur Montage, das Handbuch etc. angeordnet werden können. Das Verbindungselement 42 ist mit den Seitenelementen 36 über mit diesen verbundenen Laschen (Fig. 5) verbunden.

Zur Fixierung der Gabel des Fahrrads, weist das Bodenelement im dargestellten Ausführungsbeispiel zwei Ausnehmungen 46 (Fig. 5) auf. In die beiden Ausnehmungen 46 werden die Enden der Gabelrohre zum Transport eingesteckt. Des Weiteren weist das Bodenelement 32 des Basiselements 12 eine sich in Längsrichtung erstreckende Ausnehmung 48 (Fig. 5) auf, in der das Vorderrad 30 angeordnet wird.

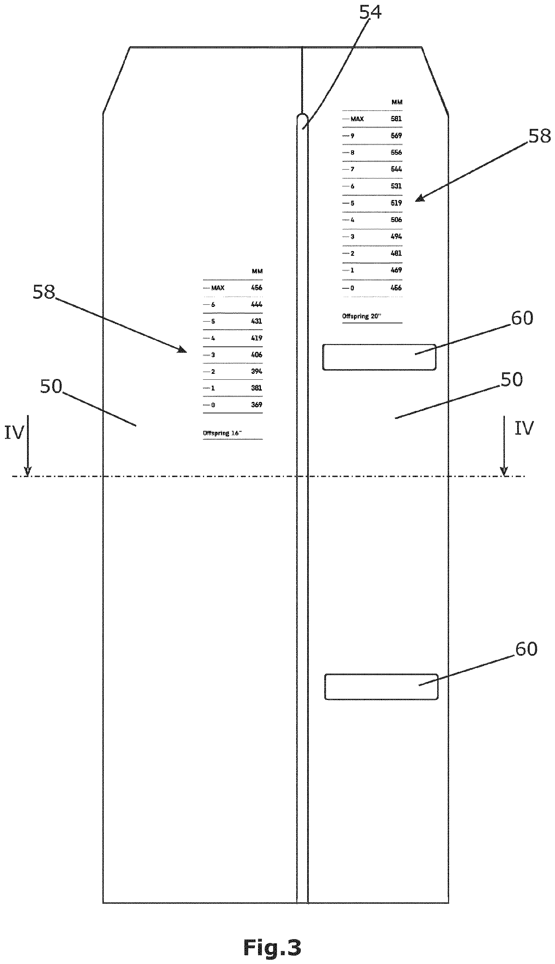

Des Weiteren ist im dargestellten Ausführungsbeispiel mit jedem der Seitenelemente 36 ein Stirnseitenelement 50 (Fig. 3) verbunden. Die beiden Stirnseiten werden zum Ausbilden einer Stirnseite des Basiselements, die in Längsrichtung 24 hinter dem Hinterrad 38 angeordnet ist, durch nach innen Klappen von Ansätzen 50 (Fig. 5) hergestellt. Die Stirnseitenelemente 50 sind ferner mit Führungslaschen 52 verbunden, die in gefaltetem Zustand nach innen weisen (Fig. 4). Zwischen den beiden Führungslaschen 52 und somit auch zwischen den beiden Stirnseitenelementen 50 ist ein vertikal verlaufender Schlitz 54 ausgebildet. Der Schlitz 54 dient zur Aufnahme bzw. Führung eines Messelements 56 (Fig. 6). Das Messelement dient zusammen mit den beiden Messskalen 58 zur Bestimmung der Sitzhöhe. Die beiden Sitzhöhenskalen 58 dienen zur Bestimmung der Sitzhöhe bei unterschiedlichen Reifendurchmessern, wobei die in Fig. 3 linke Skala für Reifendurchmesser von 16 Zoll und die rechte Skala für Reifengrößen von 20 Zoll dient, wobei selbstverständlich auch Skalen für andere Reifendurchmesser vorgesehen sein können.

Des Weiteren sind im dargestellten Ausführungsbeispiel in dem in Fig. 3 rechten Stirnseitenelement 50 zwei Grifföffnungen angeordnet. Diese dienen zum Herausziehen des Basiselements 12 aus dem Außenkarton 10 in Längsrichtung 24 (Fig. 1).

Im dargestellten Ausführungsbeispiel handelt es sich bei dem Messelement 56 nicht um ein gesondertes Element. Vielmehr wird dieses von dem Nutzer hergestellt, indem die beiden Elemente 62, 64 aus einer der beiden Seitenelemente 36 des Basiselements herausgetrennt werden. Die entsprechenden Bereiche sind perforiert, so dass auf einfache Art und Weise die Elemente 62, 64 herausgetrennt werden können. Das Element 62 wird entlang einer Mittellinie 66 zusammengefaltet. Sodann wird das Element 64 in einem rechten Winkel mittels eines Schlitzes 68 in den Schlitz 70 eingeführt. Hierdurch entsteht ein in Draufsicht von oben kreuzförmiges Element (Fig. 6). Das Element 62 weist hierbei auf der rechten Seite die beiden kleineren Ansätze auf, die einen Führungsansatz 72 ausbilden. Der in Fig. 6 linke Teil bildet einen Messansatz 74 aus. Die beiden Ansätze 72, 74 sind durch das hierzu senkrecht angeordnete Anschlagelement 76 voneinander getrennt.

Zum Bestimmen der individuellen Schritthöhe eines Benutzers zum Einstellen der Sattelhöhe wird der Führungsansatz 72 des Messelements 56 in den Schlitz 54 eingeführt, so dass eine Seite 78 des Anschlagelements 76 an einer Außenseite 80 der beiden Stirnseitenelemente 50 anliegt. Der Messansatz 74 wird zwischen den Beinen des Benutzers angeordnet. Anschließend wird das Messelement so weit wie möglich nach oben geschoben. In Abhängigkeit der Reifengröße kann die Sattelhöhe sodann unter Zuhilfenahme der Oberkante des Anschlagelements 76 auf der entsprechenden Sitzhöhenskala 58 abgelesen werden. Die Skalen weisen jeweils auf der linken Seite Ziffern auf, die beispielsweise auch auf dem Sattelstützrohr, das den Sattel trägt, vorgesehen sind. Hierdurch ist ein einfaches Einstellen der Sattelhöhe in Abhängigkeit der individuellen Schritthöhe möglich.

Zur Herstellung des Basiselements 12 wird vorzugsweise der in Fig. 5 dargestellte Kartonzuschnitt verwendet. Fig. 5 zeigt die Unterseite des entsprechenden Kartonzuschnitts. Das rechteckige Bodenelement 32 ist über Knickkanten 82 jeweils mit den beiden Seitenelementen 36 verbunden. Diese werden mit Bezug auf Fig. 5 nach hinten gekappt, so dass das Bodenelement 32 und die beiden Seitenelemente 36 einen senkrechten Winkel miteinander bilden. Ferner werden die beiden Laschen 84 wiederum um 90° nach innen geklappt, so dass die Laschen 84 parallel zu dem Bodenelement 32 sind. Die Laschen 84 bilden einerseits die Ausnehmung 48 für das Vorderrad 30 und andererseits die Ausnehmungen 46 für die beiden Gabelrohrenden aus. Zur besseren Fixierung der Gabelrohrenden in den Ausnehmungen 46 weist die in Fig. 5 obere Lasche 34 Einschnitte 86 auf.

Die beiden Stirnseitenelemente 50 werden zur Ausbildung der Stirnseite nach innen geklappt, wobei zuvor die beiden Laschen 52 wiederum um 90° nach innen geklappte werden, so dass diese in zusammengefügtem Zustand zu den Seitenelementen 36 parallel verlaufen (Fig. 4).

Fig. 7 zeigt in Unteransicht ein Halteelement 88. Bei diesem handelt es sich um einen quaderförmigen Karton. Dieser wird auf dem Lenker und dem Vorderrad 30 angeordnet. Hierzu weist das quaderförmige Halteelement 88 eine in Längsrichtung verlaufende Ausnehmung 90 zur Aufnahme des Vorderrades 30 auf, wobei die Ausnehmung 90 der Ausnehmung 48 (Fig. 5) gegenüberliegend angeordnet ist. Eine weitere Ausnehmung 92 dient zur Aufnahme des Fahrradlenkers. Das quaderförmige Halteelement 88 weist eine Breite auf, die der inneren Breite des Außenkartons 10 entspricht, so dass das Halteelement zur seitlichen Stabilisierung des Fahrradrahmens 26 sowie des Vorderrads 30 in dem Außenkarton 10 dient.

Fig. 8 zeigt schematisch eine Draufsicht eines Innenteils 40, das zwischen den beiden Seitenteilen 36 des Basiselements 12 angeordnet ist. Das Innenteil 40 weist eine in Längsrichtung 24 verlaufende Ausnehmung 94 zur Aufnahme des Hinterrads 38 auf. Das Grundelement 96 des Innenteils 40 ist rechteckig und weist eine Breite auf, die der Breite des Bodenelements 32 bzw. des Bodenteils 34 entspricht. Mit dem Grundelement 96 sind seitlich drei Laschen 98 verbunden, die als Aufstandlaschen dienen und nach unten geknickt werden. Die Laschen 98 weisen dieselbe Höhe auf wie der Bereich der Seitenteile 36 auf Höhe der Laschen 84.

## Patentansprüche

1. Fahrradtransportbehälter, insbesondere Fahrrad-Transportkarton, mit einem Außenbehälter (10), insbesondere einem Außenkarton und einem innerhalb des Außenbehälters (10) angeordneten Basiselement (12) zum Halten eines Fahrradrahmens (26), insbesondere mit montiertem Hinterrad, wobei das Basiselement (12) ein Bodenelement (32) aufweist, das auf einem Bodenteil (34) des Außenbehälters (10) aufliegt, wobei der Außenbehälter (10) ein öffenbares Seitenteil (14) aufweist, so dass das Basiselement (12) mit dem insbesondere vormontierten Fahrrad (26) seitlich aus dem Außenbehälter (10) herausziehbar ist, und
**dadurch gekennzeichnet, dass** das Basiselement (12) ein Schritthöhen-Messsystem aufweist zum Einstellen einer Sitzhöhe eines Fahrradsattels, mit
einem einen vertikal verlaufenden Schlitz (54) aufweisenden Basiselement (12),
einer zumindest auf einer Seite neben dem Schlitz (54) auf einer Oberfläche (80) des Basiselements (12) angeordneten Sitzhöhenskala (58) und
einem Messelement (56) mit einem in den Schlitz (54) einsteckbaren Führungsansatz (72) zum vertikalen Verschieben des Messelements (56) in dem Schlitz (54) und einem mit dem Führungsansatz (72) verbundenen Messansatz (74) zum Messen einer Schritthöhe.

2. Fahrradtransportbehälter nach Anspruch 1, **dadurch gekennzeichnet, dass** das Basiselement (12) durch Knicken und Zusammenfügen eines ebenen Zuschnittelements, das insbesondere ein Zuschnittkarton ist hergestellt ist.

3. Fahrradtransportbehälter nach Anspruch 1 oder 2, **dadurch gekennzeichnet, dass** das Basiselement (12) ein Bodenelement (32) und ein insbesondere zweiteilig ausgebildetes vorzugsweise mit dem Bodenelement (32) verbundenes Stirnseitenelemente (50) aufweist, wobei der Schlitz (54) in dem Stirnseitenelement (50) vorgesehen, insbesondere durch die beiden Teile des Stirnseitenelements (50) ausgebildet ist.

4. Fahrradtransportbehälter nach Anspruch 3, **dadurch gekennzeichnet, dass** das Basiselement (12) ein vorzugsweise zwei Seitenelemente (36) aufweist, die vorzugsweise mit dem Bodenelement (32) und/oder dem mindestens einen Stirnseitenelement (50) verbunden sind.

5. Fahrradtransportbehälter nach einem der Ansprüche 1 bis 4, **dadurch gekennzeichnet, dass** das Messelement (56) auf dem Basiselement (12) heraustrennbar ist.

6. Fahrradtransportbehälter nach einem der Ansprüche 1 bis 5, **dadurch gekennzeichnet, dass** das Messelement (56) zusätzlich ein Anschlagelement (76) aufweist, das vorzugsweise aus dem Basiselement (12) heraustrennbar ist.

7. Fahrradtransportbehälter nach Anspruch 6, **dadurch gekennzeichnet, dass** das Anschlagelement (76) durch Zusammenstecken mit dem Führungsansatz (72) und/oder dem Messansatz (74) verbindbar ist.

8. Fahrradtransportbehälter nach einem der Ansprüche 1 bis 7, **dadurch gekennzeichnet, dass** der Führungsansatz (72) und der Messansatz (74) einstückig ausgebildet sind.

9. Fahrradtransportbehälter nach einem der Ansprüche 1 bis 8, **dadurch gekennzeichnet, dass** das Basiselement (12) eine Ausnehmung (94) zur Aufnahme des Hinterrads und/oder eine Ausnehmung (48) zur Aufnahme eines nicht montierten Vorderrads und/oder mindestens eine Ausnehmung (46) zur Aufnahme von Gabelrohrenden aufweist.

10. Fahrradtransportbehälter nach Anspruch 9 oder 8, **dadurch gekennzeichnet, dass** das Basiselement (12) insbesondere zwei zumindest teilweise seitlich neben dem Hinterrad (32) angeordnete Seitenelemente (36) aufweist.

11. Fahrradtransportbehälter nach Anspruch 10, **dadurch gekennzeichnet, dass** die Seitenelemente (36) zur Versteifung mit zumindest einem Seitenstirnelement (50) verbunden sind.

12. Fahrradtransportbehälter nach Anspruch 11 oder 10, **dadurch gekennzeichnet, dass** die Seitenelemente (36) und vorzugsweise zusätzlich das mindestens eine Stirnseitenelement (50) mit einem oberhalb des Hinterrads (38) angeordneten Verbindungselement (42) verbunden sind.

13. Fahrradtransportbehälter nach Anspruch 9 bis 12, **gekennzeichnet durch** eine Halteelemente (88), das insbesondere im Bereich der Fahrradgabel bzw. des Fahrradlenkers angeordnet ist.

14. Fahrradtransportbehälter Anspruch 13, **dadurch gekennzeichnet, dass** das Halteelement (88) eine Ausnehmung (92) zur Aufnahme des Fahrradlenkers und vorzugsweise eine schlitzförmige Ausnehmung (90) zur Aufnahme des Vorderrads (30) aufweist.

15. Fahrradtransportbehälter nach einem der Ansprüche 9 bis 14 mit einem in dem Außenbehälter (10) angeordneten vom Basiselement (12) gehaltenen Fahrrad, insbesondere mit vormontiertem Hinterrad.

16. Verwendung des Fahrradtransportbehälters nach einem der Ansprüche 1 bis 15, zum Einstellen einer Sitzhöhe eines Fahrradsattels.

## Claims

1. A bicycle transport container, particularly a bicycle transport box, comprising
an outer container (10), particularly an outer box, and
a base element (12), arranged within the outer container (10), for holding a bicycle frame (26), particularly with the rear wheel (38) mounted,
wherein the base element (12) comprises a bottom element (32) supported on a bottom portion (34) of the outer container (10),
wherein the outer container (10) comprises an openable side portion (14) so that the base element (12) together with the preferably pre-assembled bicycle (26) can be laterally pulled out from the outer container (10), and
**characterized in that** the base element (12) comprises an inside-leg measurement system for adjusting a seat height of a bicycle saddle, comprising
a base element (12) comprising a vertically extending slot (54),
a seat height scale (58) arranged, at least on one side, laterally of the slot (54) on a surface (80) of the base element (12), and
a measuring element (56) comprising a guiding projection (72), adapted to be inserted into the slot (54), for vertical displacement of the measuring element (56) in the slot (54), and comprising a measuring projection (74), connected to the guiding projection (72), for measuring an inside leg height.

2. The bicycle transport container according to claim 1, **characterized in that** the base element (12) is produced by kinking and assembling a plane blank element which particularly is a cardboard blank.

3. The bicycle transport container according to claim 1 or 2, **characterized in that** the base element (12) comprises a bottom element (32) and an end side element (50) which is particularly of a two-part design and preferably is connected to the bottom element (32), said slot (54) being provided in the end side element (50) and particularly being formed by the two portions of the end side element (50).

4. The bicycle transport container according to claim 3, **characterized in that** the base element (12) comprises one and preferably two side elements (36) which preferably are connected to the bottom element (32) and/or to said at least one end side element (50).

5. The bicycle transport container according to any one of claims 1 to 4, **characterized in that** the measuring element (56) can be detached out from the base element (12).

6. The bicycle transport container according to any one of claims 1 to 5, **characterized in that** the measuring element (56) additionally comprises an abutment element (76) which preferably can be detached out from the base element (12).

7. The bicycle transport container according to claim 6, **characterized in that** the abutment element (76) can be connected to the guiding projection (72) and/or the measuring projection (74) by fitting the components together.

8. The bicycle transport container according to any one of claims 1 to 7, **characterized in that** the guiding projection (72) and the measuring projection (74) are formed in one piece.

9. The bicycle transport container according to any one of claims 1 to 8, **characterized in that** the base element (12) comprises a recess (94) for accommodating the rear wheel and/or a recess (48) for accommodating a non-mounted front wheel and/or at least one recess (46) for accommodating steertube ends.

10. The bicycle transport container according to claim 8 or 9, **characterized in that** the base element (12) comprises preferably two side elements (36) arranged at least partially laterally of to the rear wheel (32).

11. The bicycle transport container according to claim 10, **characterized in that**, for stiffening, the side elements (36) are connected to at least one side end element (50).

12. The bicycle transport container according to claim 10 or 11, **characterized in that** the side elements (36) and preferably additionally said at least one side end element (50) are connected to a connection element (42) arranged above the rear wheel (38).

13. The bicycle transport container according to any one of claims 9 to 12, **characterized in that** a holding element (88) is provided that is arranged preferably in the area of the bicycle fork and respectively the bicycle handlebar.

14. The bicycle transport container according to claim 13, **characterized in that** said holding element (88) comprises a recess (92) for accommodating the bicycle handlebar and preferably a slot-shaped recess (90) for accommodating the front wheel (30).

15. The bicycle transport container according to any one of claims 9 to 14, comprising a bicycle arranged in said outer container (10) and held by said base element (12), preferably with a pre-mounted rear wheel.

16. Use of a bicycle transport container according to any one of claims 1 to 15, for adjusting a seat height of a bicycle saddle.

## Revendications

1. Récipient de transport de bicyclette, en particulier carton de transport de bicyclette, doté d'un récipient externe (10), en particulier d'un carton externe et d'un élément de base (12) disposé à l'intérieur du récipient externe (10) destiné à maintenir un cadre de bicyclette (26), en particulier avec la roue arrière montée, dans lequel l'élément de base (12) comporte un élément de fond (32), lequel s'appuie sur une pièce de fond (34) du récipient externe (10), dans lequel le récipient externe (10) comporte une pièce latérale (14) pouvant s'ouvrir, de sorte que l'élément de base (12) peut être extrait latéralement hors du récipient externe (10) avec la bicyclette (26) en particulier prémontée, et
**caractérisé en ce que** l'élément de base (12) comporte un système de mesure de hauteur de marche destiné au réglage d'une hauteur d'assise d'une selle de bicyclette, doté
d'un élément de base (12) comportant une fente (54) s'étendant verticalement ;
d'une échelle de hauteur d'assise (58) disposée sur une surface (80) de l'élément de base (12) près de la fente (54) sur au moins un côté et
d'un élément de mesure (56) avec un accessoire de guidage (72) insérable dans la fente (54) pour le déplacement vertical de l'élément de mesure (56) dans la fente (54) et un accessoire de mesure (74) relié à l'accessoire de guidage (72) destiné à mesurer une hauteur de marche.

2. Récipient de transport de bicyclette selon la revendication 1, **caractérisé en ce que** l'élément de base (12) est fabriqué par pliage et assemblage d'un élément découpé plat, qui est notamment un carton découpé.

3. Récipient de transport de bicyclette selon la revendication 1 ou 2, **caractérisé en ce que** l'élément de base (12) comporte un élément de fond (32) et un élément latéral avant (50) qui est de préférence relié à l'élément de fond (32) et est en particulier réalisé en deux parties, dans lequel la fente (54) est prévue dans l'élément latéral avant (50), en particulier est formée par les deux parties de l'élément latéral avant (50).

4. Récipient de transport de bicyclette selon la revendication 3, **caractérisé en ce que** l'élément de base (12) comporte un, de préférence deux éléments latéraux, lesquels sont de préférence reliés à l'élément de fond (32) et/ou à l'au moins un élément latéral avant (50).

5. Récipient de transport de bicyclette selon l'une des revendications 1 à 4, **caractérisé en ce que** l'élément de mesure (56) sur l'élément de base (12) est détachable.

6. Récipient de transport de bicyclette selon l'une des revendications 1 à 5, **caractérisé en ce que** l'élément de mesure (56) comporte en outre un élément de butée (76), lequel est de préférence détachable de l'élément de base (12).

7. Récipient de transport de bicyclette selon la revendication 6, **caractérisé en ce que** l'élément de butée (76) peut être relié par enfichage à l'accessoire de guidage (72) et/ou à l'accessoire de mesure (74).

8. Récipient de transport de bicyclette selon l'une des revendications 1 à 7, **caractérisé en ce que** l'accessoire de guidage (72) et l'accessoire de mesure (74) sont réalisés d'une pièce.

9. Récipient de transport de bicyclette selon l'une des revendications 1 à 8, **caractérisé en ce que** l'élément de base (12) comporte un évidement (94) destiné à loger la roue arrière et/ou un évidement (48) destiné à loger une roue avant non montée et/ou au moins un évidement (46) destiné à loger des extrémités de tube de fourche.

10. Récipient de transport de bicyclette selon la revendication 9 ou 8, **caractérisé en ce que** l'élément de base (12) comporte en particulier deux éléments latéraux (36) disposés au moins en partie comme latéralement voisins de la roue arrière (32).

11. Récipient de transport de bicyclette selon la revendication 10, **caractérisé en ce que** les éléments latéraux (36) sont reliés à au moins un élément latéral avant (50) pour le renforcement.

12. Récipient de transport de bicyclette selon la revendication 11 ou 10, **caractérisé en ce que** les éléments latéraux (36) et de préférence en outre l'au moins un élément latéral avant (50) sont reliés à un élément de liaison (42) disposé au-dessus de la roue arrière (38).

13. Récipient de transport de bicyclette selon la revendication 9 à 12, **caractérisé par** un élément de maintien (88), lequel est en particulier disposé dans la zone de la fourche ou du guidon de la bicyclette.

14. Récipient de transport de bicyclette selon la revendication 13, **caractérisé en ce que** l'élément de maintien (88) comporte un évidement (92) destiné à loger le guidon de la bicyclette et de préférence un évidement en forme de fente (90) destiné à loger la roue avant (30).

15. Récipient de transport de bicyclette selon l'une des revendications 9 à 14, avec une bicyclette disposée dans le récipient externe (10) et maintenue par l'élément de base (12), en particulier avec la roue arrière prémontée.

16. Utilisation du récipient de transport de bicyclette selon l'une des revendications 1 à 15 pour le réglage d'une hauteur d'assise d'une selle de bicyclette.
